(19) Europäisches Patentamt
European Patent Office
Office européen des brevets

(11) Publication number: 0 211 670 B1

(12) # EUROPEAN PATENT SPECIFICATION

(45) Date of publication of patent specification: 09.05.90

(21) Application number: 86306134.7

(22) Date of filing: 08.08.86

(51) Int. Cl.[5]: A 61 K 31/39, A 61 K 31/425, C 07 D 327/04, C 07 D 277/14, C 07 D 277/34, C 07 D 277/36

(54) **Di-t-butylphenol compounds.**

(30) Priority: 09.08.85 US 764160
02.06.86 US 869488

(43) Date of publication of application:
25.02.87 Bulletin 87/09

(45) Publication of the grant of the patent:
09.05.90 Bulletin 90/19

(84) Designated Contracting States:
AT BE CH DE FR GB IT LI LU NL SE

(56) References cited:
EP-A-0 047 109
EP-A-0 059 090
DE-B-1 038 050

PATENT ABSTRACTS OF JAPAN, vol. 5, no. 168 (C-77)840r, 27th October 1981; & JP - A - 56 97277 (TAKEDA YAKUHIN KOGYO K.K.) 05-08-1981

(73) Proprietor: ELI LILLY AND COMPANY
Lilly Corporate Center
Indianapolis Indiana 46285 (US)

(72) Inventor: Panetta, Jill Ann
195 Scranton Court
Zionsville Indiana 46077 (US)

(74) Representative: Tapping, Kenneth George et al
Erl Wood Manor
Windlesham Surrey, GU20 6PH (GB)

(56) References cited:
CHEMICAL & PHARMACEUTICAL BULLETIN, vol. 34, no. 4, April 1986, pages 1619-1627; I. KATSUMI et al.: "Studies on styrene derivatives. II. Synthesis and antiinflammatory activity of 3,5-Di-tert-butyl-4-hydroxystyrenes"

EP 0 211 670 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to novel di-*t*-butylphenyl compounds which are useful in the treatment of antiinflammatory conditions, as well as ameliorating ischemia-induced cell damage.

Mammals, both humans and animals, are known to suffer from various conditions involving inflammation with concomitant swelling, tenderness, decreased mobility, pain, and fever. While a number of antiinflammatory agents are effective in the symptomatic treatment of such inflammatory conditions as rheumatoid arthritis, rheumatoid spondylitis, osteoarthritis, degenerative joint diseases, and the like, many such agents have a number of undesirable side effects, such as gastric irritation.

The ediology and pathogenesis of rheumatic and arthritic diseases remain obscure. Meanwhile, the need continues for safer, better calibrated drugs which will slow the process and alleviate the symptoms of inflammatory diseases. For example, in rheumatoid arthritis, any agent which reduces the inflammation is important in lessening or delaying the development of crippling.

The present invention relates to certain di-t-butylphenol derivatives which are useful as antiinflammatory agents and are also useful in slowing the development in arthritic conditions. One of the compounds employed in the method and formulations of the present invention, 5-([3,5-bis(1,1-dimethylethyl)-4-hydroxyphenyl]methylene-2-thioxo-4-thiazolidinone, is taught by Teuber *et al., Liebigs Ann. Chem.,* 757 (1978) as an intermediate in the preparation of certain compounds which are in turn used for the spin-labeling of peptides. No biological utility is disclosed for this intermediate compound. Certain 3,5-di-tert-butyl-4-hydroxyphenyl-substituted azole derivatives are taught to be antiinflammatory agents by Isomura *et al., Chem. Pharm. Bull. 32,* (1), 152 (1984).

The antiinflammatory activity of α-(3,5-di-tert-butyl-4-hydroxybenzylidene)-γ-butyrolactone is taught by Hidaka *et al., Japan, J. Pharmacol., 36,* 77 (1984).

Certain rhodanine derivatives in U.S. Patent No. 4,464,382 are claimed to be inhibitors of aldose reductase. However, the claimed compounds all require acetic acid substituents on the nitrogen atom of the rhodanine ring.

In accordance with the invention it has been found that di-t-butylphenol compounds of the formula I:

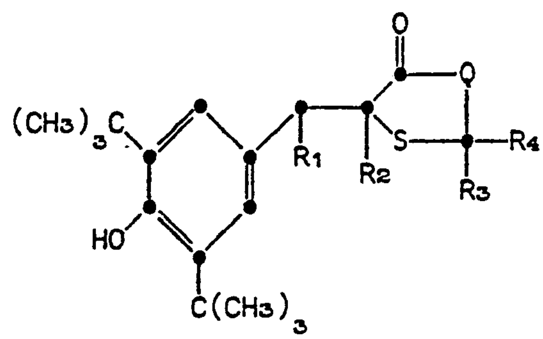

I

wherein Q is O or NR, where R is hydrogen, or $C_1$—$C_4$ alkyl;

$R_1$ and $R_2$ are each hydrogen, or when taken together form a bond; and

$R_3$ and $R_4$ are each hydrogen, or when taken together are =S, provided that when $R_1$ and $R_2$ are a bond, Q is NR and R=H, then $R_3$ and $R_4$ are each H and their pharmaceutically-acceptable salts, are useful in the treatment of antiinflammatory conditions such as arthritis.

According to a further aspect of the present invention there is provided a pharmaceutical formulation which comprises as an active ingredient a compound of formula I as defined above, or a pharmaceutically-acceptable salt thereof, associated with one or more pharmaceutically acceptable carriers or excipients therefor.

The compounds of formula I, except that wherein $R_1$ and $R_2$ taken together represent a bond, Q is NH and $R_3$ and $R_4$ taken together represent =S, and their pharmaceutically-acceptable salts are novel and are provided in yet a further aspect of the invention.

Moreover, compounds of formula III;

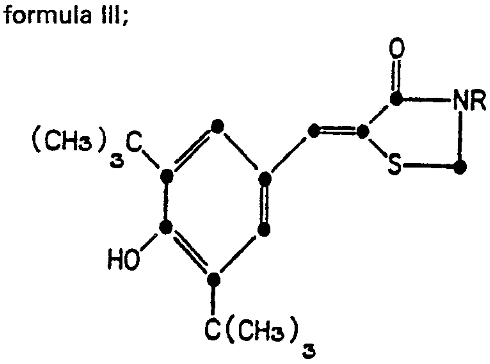

III

where R is hydrogen or $C_1$—$C_4$ alkyl, and their pharmaceutically-acceptable salts, are also useful for reversing ischemia-induced cell damage in mammals, particularly brain damage caused by stroke.

The compounds of formula I include 5-{[3,5-bis(1,1-dimethylethyl)-4-hydroxyphenyl]methylene}-2-thioxo-4-thiazolidinone (referred to hereinafter as compound IIa), 5-{[3,5-bis(1,1-dimethylethyl)-4-hydroxyphenyl]methylene}-4-thiazolidinone (compound IIb), 5-{[3,5-bis(1,1-dimethylethyl)-4-hydroxyphenyl]methyl}-4-thiazolidinone (compound IIc), 5-{[3,5-bis(1,1-dimethylethyl)-4-hydroxyphenyl]methyl}-2-thioxo-4-thiazolidinone (compound IId) and 5-{[3,5-bis(1,1-dimethylethyl)-4-hydroxyphenyl]methylene}-3-methyl-4-thiazolidinone (compound IIe). The compounds of formula I include compounds IIb, IIc, and IId. The compounds wherein $R_1$ and $R_2$ taken together form a bond (compounds IIa, IIb and IIe) are preferred, with compound IIe being most preferred.

Other compounds are similarly named. Compounds of Formula I wherein Q is O and $R_3$ and $R_4$ are =S are known as 2-thioxo-1,3-oxathiolan-5-ones whereas the corresponding desthione derivatives ($R_3$ and $R_4$ are each hydrogen) are 1,3-oxathiolan-5-ones. The thiazolidinones (Q is NR) are preferred, especially those wherein R is hydrogen or methyl.

The term "$C_1$—$C_4$ alkyl" refers to methyl, ethyl, propyl, isopropyl, butyl, isobutyl, sec-butyl, and tert-butyl.

Compound IIa is taught in the art by Teuber *et al., Leibigs Ann. Chem.,* 757 (1978). The compound is prepared by the reaction of 3,5-di-tert-butyl-4-hydroxybenzaldehyde with rhodanine at reflux in glacial acetic acid with fused sodium acetate as a catalyst.

Compounds IIb, IIc, and IId can be prepared from compound IIa. For example, when compound IIa is subjected to catalytic hydrogenation, one obtains both IIa and IIc. The relative proportions of each depends upon the temperature, pressure, and duration of hydrogenation, the solvent employed, and the particular catalyst used. For example, when compound IIa is treated with 5% palladium on carbon in ethanol at 100°C for approximately 18 hours, the relative ratios of IIb/IIc are approximately 60:40. Alternatively, these transformations may be accomplished by heating compound IIa in a mixture of hydrochloric acid and an alcohol such as ethanol in the presence of zinc. Reduction of the thione without affecting the benzylic double bond may be accomplished by heating the thione with a reducing agent such as a trialkyltin hydride, particularly tri-n-butyltin hydride, in a non-reactive solvent, such as toluene, and preferably in the presence of a free radical initiator, such as azobisisobutyronitrile. The reaction can typically be accomplished using temperatures in the range from 50° to 120°C.

The transformation of compound IIa to IId may be accomplished by a variety of methods known in the art. A preferred method is that taught by Nakamura *et al., Tetrahedron Letters, 25,* 3983 (1984). In this reaction, compound IIa is treated with a dihydropyridine such as diethyl 2,6-dimethyl-1,4-dihydro-3,5-pyridine-dicarboxylate in the presence of silica gel. The reaction is best carried out in the presence of a non-reactive solvent such as benzene or toluene, preferably under an inert atmosphere. The reaction may be accomplished at temperatures from about 25°C up to the reflux temperature of the mixture. At the preferred temperature of approximately 80°C, the reaction is essentially complete after 12—18 hours.

Other thiazolidinones may be prepared in similar fashion. For example, compounds of Formula II wherein Q is NR and R is $C_1$—$C_4$ alkyl may be prepared according to the above methods, initially by the analogous reaction taught for IIa employing the appropriate N-substituted rhodanine. Alternatively, an unsubstituted compound (II, Q=NH) may be alkylated with the appropriate alkylating agent of formula RL where L is an appropriate leaving group such as halo, e.g. the iodide or bromide, to provide the corresponding N-alkylated derivatives of Formula II. Such a transformation is usually accomplished in an inert solvent such as tetrahydrofuran or dimethylformamide, and in the presence of a strong base, such as sodium hydride. Typical reaction temperatures lie within the range 0° to 100°C.

The corresponding 1,3-oxathiolan-5-ones may be prepared from β-(3,5-di-t-butyl-4-hydroxyphenyl)-α-mercaptoacrylic acid (IV). Compound IV may be treated with carbon disulfide to prepare the thione analog (II, Q=O, $R_3$ and $R_4$ are =S), while reaction of IV with formic acid provides the corresponding desthione (II, Q=O, $R_3$ and $R_4$ are each hydrogen). Compound IV can be prepared by known methods (see, eg, Campaigne *et al., J. Org. Chem., 26,* 359 (1961); *id., 26,* 1326 (1961); Chakrabarti, et al, *Tetrahedron, 25* (14), 2781 (1969)), or upon heating compound IIa with dilute aqueous base (See Example 10A).

According to one aspect of the invention there is provided a process for preparing a novel compound of formula I which comprises

(A) reacting a compound of formula:

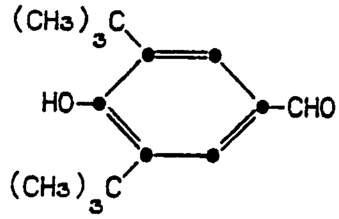

with a compound of formula

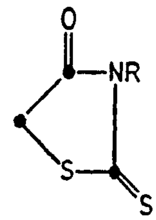

so as to provide a compound of formula I wherein $R_1$ and $R_2$ taken together represent a bond, Q is NR, and $R_3$ and $R_4$ taken together represent =S;

(B) reducing a compound of formula I wherein $R_3$ and $R_4$ represent =S so as to prepare a compound of formula I in which $R_3$ and $R_4$ are hydrogen;

(C) reducing a compound of formula I in which $R_1$ and $R_2$ represent a bond so as to prepare a compound of formula I in which $R_1$ and $R_2$ are hydrogen;

(D) alkylating a compound of formula I in which R is hydrogen so as to prepare a compound of formula I in which R is $C_1$—$C_4$ alkyl; or

(E) reacting a compound of formula

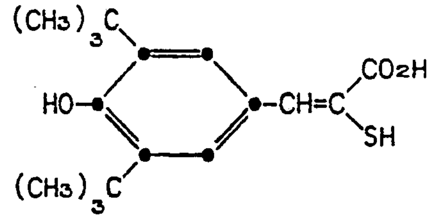

with

(i) formic acid so as to provide a compound of formula I wherein Q is O, $R_1$ and $R_2$ taken together represent a bond, and $R_3$ and $R_4$ represent hydrogen; or

(ii) carbon disulfide, so as to provide a compound of formula I wherein Q is O, $R_1$ and $R_2$ taken together represent a bond, and $R_3$ and $R_4$ taken together represent =S.

Depending upon the definitions of $R_1$ and $R_2$, the compounds of formula I may exist in various isomeric forms. This invention is not limited to any particular isomer but includes all possible individual isomers and racemates. Pharmaceutically-acceptable salts can be prepared by reacting a compound of formula I with a strong base, such as sodium hydroxide.

The following examples further illustrate the preparation of the compounds of this invention.

### Example 1

5-{[3,5-bis(1,1-dimethylethyl)-4-hydroxyphenyl]methylene}-2-thioxo-4-thiazolidinone (compound IIa)

Under a nitrogen atmosphere, 117.2 g of 3,S-di-tert-butyl-4-hydroxybenzaldehyde, 66.6 g of rhodanine, and 143.5 g of fused sodium acetate were heated at reflux in 2500 ml of glacial acetic acid. After heating for 23 hours, the reaction mixture was cooled and poured into a mixture of 1 liter of ethanol and 1 liter of ice with stirring. Five hundred milliliters of water were added and, after stirring for 30 minutes, the resulting precipitate was recovered by filtration. The solid was slurried with 500 ml of ethyl acetate and filtered. The precipitate was then dissolved in 3 liters of ethanol, heated to boiling, and water was added until the solution remained cloudy, approximately 450 ml. Upon cooling to room temperature, 99.6 g of the desired title product were recovered by filtration, m.p. approximately 260°C.

Analysis for $C_{18}H_{23}NO_2S_2$

| | | | |
|---|---|---|---|
| Calculated: | C, 61.86; | H, 6.63; | N, 4.01; |
| Found: | C, 62.13; | H, 6.55; | N, 4.15 |

### Examples 2—3

5-{[3,5-bis(1,1-dimethylethyl)-4-hydroxyphenyl]methylene}-4-thiazolidinone (compound IIb) and 5-{[3,5-bis(1,1-dimethylethyl)-4-hydroxyphenyl]methyl}-4-thiazolidinone (compound IIc)

A solution of 69.90 g of 4-{[3,5-bis(1,1-dimethylethyl)-4-hydroxyphenyl]methylene}-2-thioxo-4-thiazolidinone in 4 liters of ethanol was hydrogenated at 500 psi in the presence of 200 g of 5% palladium on carbon overnight at 100°C. The reaction mixture was filtered and evaporated to dryness. In sections the material was dissolved in 1 volume of hot ethyl acetate, diluted with 2 volumes of hexane, filtered, and loaded onto a silica gel chromatography column. Elution with 35% ethyl acetate in hexane provided various fractions which were combined according to the purities of the respective compounds. A total of 4.6 g of compound IIb were isolated by chromatography. Fractions which were predominantly compound IIb were crystallized from ethyl acetate/hexane providing a total yield of compound IIb of 13.79 g. Rechromatography of fractions containing impure compound IIc on silica eluting with 25% ethyl acetate in hexane provided 9.82 g of compound IIc.

2. 5-{[3,5-bis(1,1-dimethylethyl)-4-hydroxyphenyl]methylene}-4-thiazolidinone, m.p. 209—213°C.

Analysis for $C_{18}H_{25}NO_2S$

Calculated: C, 67.67; H, 7.89; N, 4.38;

Found: C, 67.44; H, 8.11; N, 4.65.

3. 5-{[3,5-bis(1,1-dimethylethyl)-4-hydroxyphenyl]methyl}-4-thiazolidinone, m.p. 149—152°C.

Analysis for $C_{18}H_{27}NO_2S$

Calculated: C, 67.25; H, 8.47; N, 4.36;

Found: C, 67.43; H, 8.44; N, 4.21.

Example 4

5-{[3,5-bis(1,1-dimethylethyl)-4-hydroxyphenyl]methyl}-2-thioxo-4-thiazolidinone (compound IId)

Under a nitrogen atmosphere, 13.98 g of 5-{[3,5-bis(1,1-dimethylethyl)-4-hydroxyphenyl]methylene}-2-thioxo-4-thiazolidinone, 13.17 g of diethyl 2,6-dimethyl-1,4-dihydro-3,5-pyridinedicarboxylate and 600 ml of toluene were stirred to effect solution. Forty grams of silica gel 60 (finer than 230 mesh) previously dried *in vacuo* at 50°C for 7 hours were added to the reaction. The reaction was heated at reflux for 18 hours and filtered hot. The filtrate was evaporated to dryness. The residue was dissolved in 500 ml of ethyl acetate, washed 5 times each with 400 ml of 1N hydrochloric acid, dried over sodium sulfate, filtered, and evaporated *in vacuo* to provide a yellow solid. Chromatography over silica gel eluting with 2.5% ethyl acetate in toluene provided 8.0 g of the desired title product, m.p. 178—179°C.

Analysis for $C_{18}H_{25}NO_2S_2$

Calculated: C, 61.50; H, 7.17; N, 3.98;

Found: C, 61.28; H, 7.19; N, 3.94

Example 5

5-{[3,5-bis(1,1-dimethylethyl)-4-hydroxyphenyl]methylene}-3-methyl-2-thioxo-4-thiazolidinone

The title compound was prepared in 76% yield from 3,5-di-tert-butyl-4-hydroxybenzaldehyde and N-methylrhodamine following the procedure of Example 1, m.p. >260°C.

Analysis for $C_{19}H_{25}NO_2S_2$

Calculated: C, 62.77; H, 6.93; N, 3.85; S, 17.64;

Found: C, 62.54; H, 7.05; N, 3.66; S, 17.82.

Example 6

5-{[3,5-bis(1,1-dimethylethyl)-4-hydroxyphenyl]methylene}-3-methyl-4-thiazolidinone

The title compound was prepared in 71% yield from 10.31 g of the thione of Example 5 upon heating with 38.15 ml of tri-n-butyltin hydride and 1.16 g of azobisisobutyronitrile (AIBN) in 142 ml of toluene at reflux for one hour. The product was isolated by adding water to the cooled reaction mixture, separating the layers, washing the organic layer with 1N hydrochloric acid and a saturated sodium chloride solution, drying over magnesium sulfate, concentrating *in vacuo,* and purifying the residue by chromatography over silica gel eluting with a 10—50% hexane in ethyl acetate gradient. The purified product had a melting point of 142—144°C.

Analysis for $C_{19}H_{27}NO_2S$

Calculated: C, 68.43; H, 8.16; N, 4.20;

Found: C, 68.68; H, 8.00; N, 3.97.

Example 7

5-{[3,5-Bis(1,1-dimethylethyl)-4-hydroxyphenyl]methylene}-3-ethyl-4-thiazolidinone

To a solution of 9.58 g of 5-{[3,5-bis(1,1-dimethylethyl)-4-hydroxyphenyl]methylene}-4-thiazolidinone in 150 ml of tetrahydrofuran were added 1.20 g of a 60% dispersion of sodium hydride in mineral oil. After gas evolution ceased, 2.4 ml of ethyl iodide were added and the reaction mixture was stirred two days under an argon atmosphere. The mixture was heated at reflux for six hours, cooled, diluted with diethyl ether and water, and adjusted to pH 3 with 1N hydrochloric acid. The layers were separated, and the organic layer was washed with a saturated sodium bicarbonate solution followed by a saturated sodium chloride solution. Concentration of the dried organic solution and chromatography of the resulting residue over silica gel eluting with a 10—30% ethyl acetate in hexane gradient provided 3.65 g of the desired title product, m.p. 169—172.5°C.

Analysis for $C_{20}H_{29}NO_2S$

Calculated: C, 69.12; H, 8.41; N, 4.03;

Found: C, 69.39; H, 8.52; N, 4.30.

Examples 8—9

The following compounds were prepared from the appropriate alkyl iodide according to the procedure of Example 7.

8. 5-{[3,5-bis(1,1-dimethylethyl)-4-hydroxyphenyl]methylene}-3-propyl-4-thiazolidinone, 60% yield, m.p. 145—146.5°C.

Analysis for $C_{21}H_{31}NO_2S$

Calculated: C, 69.76; H, 8.64; N, 3.87;
Found: C, 70.05; H, 8.76; N, 4.01.

9. 5-{[3,5-bis(1,1-dimethylethyl)-4-hydroxyphenyl]methylene}-3-butyl-4-thiazolidinone, 60% yield, m.p. 168.5—169.5°C.

Analysis for $C_{22}H_{33}NO_2S$
Calculated: C, 70.36; H, 8.86; N, 3.73;
Found: C, 70.60; H, 8.81; N, 3.97.

Example 10

4-{[3,5-Bis(1,1-dimethylethyl)-4-hydroxyphenyl]methylene}-1,3-oxathiolan-5-one

A. Preparation of β-(3,5-di-t-butyl-4-hydroxyphenyl)-α-mercaptoacrylic acid.

A solution of 174.5 g of 5-{[3,5-bis(1,1-dimethylethyl)-4-hydroxyphenyl]methylene}-2-thioxo-4-thiazolidinone in 1250 ml of a 10% sodium hydroxide solution was heated on a steam bath for four hours. Decolorizing carbon was added and the mixture filtered through a diatomaceous earth pad. The filtrate was chilled by adding ice and treated with 6N hydrochloric acid. The precipitated product was recovered by filtration, washed with water, and dried providing 150 g. of the desired subtitled intermediate.

B. Preparation of 4-{[3,5-bis(1,1-dimethylethyl)-4-hydroxyphenyl]methylene}-1,3-oxathiolan-5-one.

Following the procedure of *Agr. Biol. Chem., 29* (8), 728 (1965), six grams of the mercaptoacrylic acid from above were heated on a steam bath with 36 ml of acetic acid and 6 ml of formaldehyde (37% solution) for one hour. Evaporation of the mixture and chromatography of the residue over silica gel provided 1.7 g of the desired product, m.p. 127—129°C.

Analysis for $C_{18}H_{24}O_3S$
Calculated: C, 67.47; H, 7.55;
Found: C, 67.71; H, 7.62.

The compounds of formula I have been found to inhibit 5-lipoxygenase *in vitro.* In addition, the compounds have been shown to be inhibitors of phospholipase $A_2$. Moreover, the compounds have demonstrated activity *in vivo* in various test systems designed to detect effective antiinflammatory and antiarthritic agents. These pharmacodynamic effects were demonstrated in the following test systems.

Carrageenin Assay

The compounds were evaluated for antiinflammatory activity in the test method described by C.A. Winter, *Proc. Soc. Exp. Biol. Med., 111,* 544 (1962). In this test, inflammation is created by injecting carrageenin into the hind paws of rats. Test compounds are administered prior to injection to determine percent inhibition of the subsequent inflammation in comparison with control animals. The results are reported in Table I.

TABLE I

Antiinflammatory Activity in the Carrageenin Assay

| Compound of Example No. | Dose mg/kg* | Percent Inhibition |
|---|---|---|
| 1 | 30 | 67% |
| 2 | 50 | 46% |
| 3 | 50 | 12% |
| 4 | 50 | 65% |

* orally by gavage

Antigen-Induced Granulomatous Inflammation In Guinea Pig

Following the general procedure of Andersson, *Br. J. Pharmac., 69,* 467 (1980), mixed sex Hartley strain guinea pigs (250—300 g) were sensitized to ovalbumin by a single injection of 1 mcg of ovalbumin mixed with 50 mg of aluminum hydroxide per animal. These animals were used 21 to 26 days later for injection of ovalbumin covalently bound to agarose into the foot pad.

Granulomatous inflammation was induced by injection of 0.1 ml of an Affi-Gel Ovalbumin (Bio-Rad Laboratories, Richmond, California) into the foot pad of the sensitized guinea pigs. Three days after the injection, the volume of the injected and uninjected foot pads were measured. Paw (foot pad) volumes were measured by water displacement using a Statham pressure transducer and digital voltmeter. The difference in paw volumes between the injected and uninjected paw represents the degree of inflammation. Histological examinations of the inflamed paws show the infiltration of neutrophils between day one and three, and macrophages by day three. By day 11, marked focally extensive granulomatous

inflammation is observed. A marked inflammatory cell infiltrate consisting primarily of macrophages with lesser numbers of multinucleated giant cells, eosinophils, plasma cells and lymphocytes surrounds the Affi-Gel Ovalbumin spheres.

Groups of five guinea pigs which have received injection of the Affi-Gel Ovalbumin in the foot pad three days previously, were treated with the test compounds, clinical anti-rheumatic drugs such as penicillamine, or placebo mixed in the diet for eight days or orally by gavage for four days. The paw volumes were measured and the difference in volumes between the injected and uninjected paws was then compared to the difference on day three, the day drug treatment was initiated. The percent inhibition of granulomatous inflammation was determined by the following formula:

$$\% \text{ inhibition} = \left(1 - \frac{\text{Diffvol-11}}{\text{Diffvol-3}}\right) \times 100$$

where Diffvol-3 is the difference in paw volumes between injected and uninjected paws on day three and Diffvol-11 is the difference in paw volumes between injected and uninjected paws on day eleven. The results of these studies are summarized in Table II.

TABLE II

Antiinflammatory Activity in the Antigen-induced Granulomatous Assay

| Compound Tested | Dose/Route of Administration | Percent Inhibition |
|---|---|---|
| 1 | 0.1% in diet | 64% |
| | 25 mg/kg P.O. | 5% |
| | 50 mg/kg P.O. | 23% |
| | 100 mg/kg P.O. | 55% |
| 2 | 0.1% in diet | 61% |
| 3 | 0.1% in diet | 67% |
| 4 | 0.1% in diet | 5% |
| Azathioprine | 50 mg/kg P.O. | 57% |
| D-Penicillamine | 100 mg/kg P.O. | 60% |
| | 200 mg/kg P.O. | 73% |
| Acetaminophen | 100 mg/kg P.O. | 69% |
| Dexamethazone | 3 mg/kg P.O. | 62% |
| | 1 mg/kg P.O. | 70% |

Collagen-Induced Arthritis Assay

Type II collagen was isolated from bovine articular cartilage by the method of Strawich and Nimni [*Biochemistry, 10,* 3905 (1971)]. The collagen was dissolved in 0.1 M acetic acid and stored at −20°C. Type II collagen solution was diluted to 2 mg/ml concentration and emulsified thoroughly with an equal volume of incomplete Freund's adjuvant (ICFA). The emulsion containing approximately 0.5 mg of collagen was injected intradermally on day 0 to groups of 6 inbred Lewis male rats (Charles River Breeders; 170—200 g) at various sites in the dorsal area. The hindpaw volumes of each rate were measured and recorded three times a week throughout the test period to assess the inflammatory reaction. The test group animals received compounds under test as suspensions in carboxymethylcellulose vehicle, by oral gavage, 5 days per week (Monday-Friday), beginning on day 1. Control animals received vehicle without a test compound. At the end of the test (day 28 or 30), the blood of these animals was drawn by cardiac puncture and the serum anti-type II collagen antibody levels were estimated by passive hemagglutination technique, using glutaraldehye treated sheep red cells, to which type II collagen is conjugated [Avrameas et al., *Immuno-*

*chemistry, 6,* 67 (1969); [Andriopoulos et al., *Arth. Rheum., 19,* 613 (1976)]. The cellular response or delayed-type hypersensitivity response to type II collagen was measured by the radiometric ear index assay [Kostiala, *Immunology, 33,* 561 (1977)]. In certain experiments, the bone damage occurring because of immunization with type II collagen and the effects of drugs were determined from the radiographs of the hindpaws of two or three representative animals from each group. Injections of ICFA without collagen II were employed in some rats as a negative control; these rats received only carbomethoxycellulose vehicle during the test.

The results of testing the compounds of Formula I in the collagen-induced arthritis system are summarized in Table III. The % inhibition was calculated according to the following formula:

$$\% \text{ inhibition} = [1 - \frac{Vt - Vv}{Vc - Vv}] \times 100$$

where Vt is the hindpaw volume of a compound-treated animal (test group), Vc is the hindpaw volume of a non-compound-treated animal (carbomethoxycellulose vehicle only-the control group), and Vv is the hindpaw volume of a vehicle (carbomethoxycellulose) treated animal which received ICFA with no collagen II (negative control group).

TABLE III

Inhibition of Collagen-Induced Arthritis

| Compound of Example No. | Dose mg/kg* | % inhibition* |
|---|---|---|
| 1 | 50 | 100% |
| | 50 | 53% |
| 2 | 30 | 91% |
| | 50 | 100% |
| | 30 | 50% |
| 3 | 50 | 79% |
| 4 | 50 | 5% |

* See text for experimental method.

Developing Adjuvant-induced arthritis test in rats

Compounds were tested for their ability to alter hind paw swelling and bone damage resulting from adjuvant-induced edema in rats. In order to quantitate the inhibition of hind paw swelling resulting from adjuvant-induced arthritis, two phases of inflammation have been defined: (1) the primary and secondary *injected* hind paw, and (2) the secondary *uninjected* hind paw, which generally begins developing about eleven days from the induction of inflammation in the injected paw. Reduction of the latter type of inflammation is an indication of immunosuppressive activity. Cf. Chang, *Arth. Rheum., 20,* 1135—1141 (1977).

Adjuvant arthritis was induced in male Lewis-Wistar rats (200—210 grams) by a single subplantar injection into the right hind paw of 0.1 ml of a 0.5% suspension of heat-killed, lyophilized *Mycobacterium tuberculosis* (Calbiochem-Perrigen-C) in mineral oil (a modification of a method reported by Winter *et al., Arth. Rheum., 9,* 394—397 (1966)). One group of 10 rats ("TB control") received only this treatment. Another group of 5 rats received no treatment (normal control). Each compound to be tested was suspended in carboxymethylcellulose (1%) and administered p.o. to rats (groups of 5 each) in daily doses of 50 mg/kg beginning on day one and continuing through the 28th day after the adjuvant injection (29 doses). Paw volumes were measured by mercury displacement using a Statham pressure transducer and digital voltmeter. Volumes of both the injected and the uninjected hind paws were measured on days 16, 18, 21, 23, 25, 28, and 30. X-ray photos were taken on day 30, after the animals were sacrificed. The paw volume measurements on the uninjected paw beginning with day 16 through day 30 were computer plotted for the TB controls, the normal controls, and the drug-treated animals, and the areas under the curves [(TB controls minus normal controls) and (treated animals minus normal controls)] were determined. The results are summarized in Table IV.

TABLE IV

Inhibition of Uninjected Paw Volume Inflammation

Days 16 through 30

| Compound of Example No. | Dose mg/kg P.O. × 29 | % Inhibition* |
|---|---|---|
| 1 | 50 | 40% |
| 2 | 50 | 52% |
| 3 | 50 | 10% |
| 4 | 50 | 4% |
| 6 | 50 | 57% |
| 7 | 50 | 6% |
| 8 | 50 | 40% |
| 10 | 50 | 52% |

* % inhibition is the difference of the areas under the curves (AUC) of the mean uninjected paw volumes plotted for days 16, 18, 21, 23, 25, 28 and 30 according to the following formula:

$$\% \text{ inhibition} = [1 - \frac{\text{(Drug treated AUC)-(normal control AUC)}}{\text{(TB control AUC)-(normal control AUC)}}] \times 100$$

Compounds of Formula III have also been shown to prevent ischemia-induced cell damage particularly neuronal cell damage caused by stroke as demonstrated in the following test system.

Stroke model in rats

Strokes were produced in rats by occluding the four arteries that supply blood to the brain according to the following procedure: Male Wistar rats were anesthetized with Metofane and placed into a stereotaxic instrument. A longitudinal incision was made on the dorsal surface of the neck. The neck muscles were reflected to expose the dorsal surface of the spinal column. The two vertebral arteries were exposed where they pass through the first cervical vertebra. Both arteries were permanently occluded by the application of electrocautery. After coagulation of the vertebral arteries, the rat was removed from the stereotaxic instrument and the surgical wound was sutured. Two longitudinal incisions were then made on the ventral surface of the neck. The two common carotid arteries were exposed and dissected free from surrounding nerves and connective tissue. An atraumatic clasp, fabricated mainly from silicon rubber tubing, was placed around each carotid artery in a manner such that the vessel was not traumatized or occluded. The surgical wounds were then closed. The atraumatic clasps were designed in such a manner that they could be tightened to occlude the carotid arteries by pulling on a small silastic thread that was allowed to protrude from the wound. Circulation to the brain through the carotids could be restored by relieving the tension on the silastic threads. After the surgery, the rats were allowed to recover for 24 hours.

On the day of testing, compounds were suspended in 2% acacia and were administered orally at various times before stroke induction. Strokes (cerebral ischemia) were induced by tightening the clasps around the carotids for a period of 30 minutes. During this time, rats in which strokes had successfully been produced lost the righting reflex and became unresponsive to stimuli. After 30 minutes of ischemia, tension on the clasps was removed and blood flow to the brain restored. Rats were again treated with compounds on the morning after the stroke. On the third day after the stroke the animals received an overdose of barbituate anesthetic, and the brain was perfused *in situ* with 10% neutral, buffered formalin. After perfusion with amounts of formalin adequate to fix the brain, the brains were removed and stored in 10% formalin until histological sections could be prepared.

One of the areas of the brain that is most susceptible to ischemia induced damage both in the rat and the human is the $CA_1$ pyramidal cell layer of the hippocampus. In animals that remain unresponsive for the 30 minute period of ischemia, the $CA_1$ pyramidal cell layer is completely destroyed. This layer of cells was examined microscopically in histological sections prepared from the hippocampus. Brain damage was rated according to the following scale:

0 = no damage, completely intact cell layer
1 = mild damage, one-third of $CA_1$ layer dead
2 = moderate damage, two-thirds of $CA_1$ layer dead
3 = severe damage, complete destruction of $CA_1$ layer

Damage in 10—12 sections from each brain was assessed in order to obtain an accurate estimate of damage. An average damage score was calculated for each treatment group. Scores from treated groups were compared statistically with scores from control groups which received only the vehicle (2% Acacia) that was used to suspend the compounds. The level of significance was determined using Student's "t-test". Results are summarized in Table V.

TABLE V

Prevention of ischemia-induced brain damage in the hippocampal $CA_1$ region in rats

| Treatment | Dose* | No. of Rats | Damage Score** |
|---|---|---|---|
| Vehicle control | — | 6 | $2.5 \pm 0.2$ |
| Example 2 | 50 | 10 | $1.2 \pm 0.2^{+}$ |
| Vehicle control | — | 4 | $2.3 \pm 0.8$ |
| Example 2 | 200 | 6 | $0.2 \pm 0.2^{+}$ |
| Vehicle control | — | 10 | $2.5 \pm 0.2$ |
| Example 2 | 500 | 7 | $0.07 \pm 0.007^{++}$ |
| Vehicle control | — | 3 | $2.8 \pm 0.2$ |
| Example 2 | 500 | 4 | $0.0^{++}$ |
| Vehicle control | — | 9 | $2.4 \pm 0.4$ |
| Example 6 | 50 | 6 | $0.7 \pm 0.3^{++}$ |
| Vehicle control | — | 4 | $1.5 \pm 0.5$ |
| Example 6 | 150 | 4 | $0.3 \pm 0.3$*** |

* mg/kg gives orally as a suspension in 2% acacia
** mean ± standard error
***Three rats showed no damage, one rat died and was revived; $p<0.02$.
$^{+}$ $p<0.02$; $^{++}p<0.001$

The compounds of Formula II are antiinflammatory and antiarthritic agents and the invention provides such methods. The methods comprise administering a compound of Formula II by various routes including the oral, rectal, transdermal, subcutaneous, intravenous, intramuscular, or intranasal routes, being usually employed in the form of a pharmaceutical composition. It is a special feature of these compounds that they are effective following oral administration. Such compositions are prepared in a manner well known in the pharmaceutical art and comprise at least one active compound.

In making the compositions of the present invention, the active ingredient will usually be mixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin capsules, suppositories, sterile injectable solutions and sterile packaged powders.

Some examples of suitable carriers, excipients, and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, methyl cellulose, methyl- and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient by employing procedures well known in the art.

The compositions are preferably formulated in a unit dosage form, each dosage containing from about 5 to about 500 mg, more usually about 25 to about 300 mg, of the active ingredient. The term "unit dosage

form" refers to physically discrete units suitable as unitary dosages for human subjects and other mammals, each unit containing a predetermined quantity of active material calculated to produce the desired therapeutic effect, in association with a suitable pharmaceutical carrier.

The active compounds are effective over a wide dosage range. For example, dosages per day will normally fall within the range of about 0.5 to about 200 mg/kg of body weight. In the treatment of adult humans, the range of about 1 to about 50 mg/kg, in single or divided doses, is preferred. However, it will be understood that the amount of the compound actually administered will be determined by a physician, in the light of the relevant circumstances including the condition to be treated, the choice of compound to be administered, the chosen route of administration, the age, weight, and response of the individual patient, and the severity of the patient's symptoms, and therefore the above dosage ranges are not intended to limit the scope of the invention in any way.

The following formulation examples may employ as active compounds any of the compounds of Formula I. The examples are illustrative only and are not intended to limit the scope of the invention in any way.

Example 11

Hard gelatin capsules are prepared using the following ingredients:

| | Quantity (mg/capsule) |
|---|---|
| 5-{[3,5-bis(1,1-dimethylethyl)-4-hydroxyphenyl]-methyl}-2-thioxo-4-thiazolidinone | 250 |
| Starch dried | 200 |
| Magnesium stearate | 10 |

The above ingredients are mixed and filled into hard gelatin capsules in 460 mg quantities.

Example 12

A tablet formula is prepared using the ingredients below:

| | Quantity (mg/tablet) |
|---|---|
| 5-{[3,5-bis(1,1-dimethylethyl)-4-hydroxyphenyl]-methylene}-4-thiazolidinone | 250 |
| Cellulose, microcrystalline | 400 |
| Silicon dioxide, fumed | 10 |
| Stearic acid | 5 |

The components are blended and compressed to form tablets each weighing 665 mg.

Example 13

An aerosol solution is prepared containing the following components:

| | Weight % |
|---|---|
| 5-{[3,5-bis(1,1-dimethylethyl)-4-hydroxyphenyl]methyl}-4-thiazolidinone | 0.25 |
| Ethanol | 29.75 |
| Propellant 22 (Chlorodifluoromethane) | 70.00 |

The active compound is mixed with ethanol and the mixture added to a portion of the propellant 22, cooled to −30°C and transferred to a filling device. The required amount is then fed to a stainless steel container and diluted with the remainder of the propellant. The valve units are then fitted to the container.

Example 14

Tablets each containing 60 mg of active ingredient are made up as follows:

| | |
|---|---|
| 5-{[3,5-bis(1,1-dimethylethyl)-4-hydroxyphenyl]-methylene}-2-thioxo-4-thiazolidinone | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50—60°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

Example 15

Capsules each containing 80 mg of medicament are made as follows:

| | |
|---|---|
| 4-{[3,5-bis(1,1-dimethylethyl)-4-hydroxyphenyl]methyl} 1,3-oxothiolan-5-one | 80 mg |
| Starch | 59 mg |
| Microcrystalline cellulose | 59 mg |
| Magnesium stearate | 2 mg |
| Total | 200 mg |

The active ingredient, cellulose, starch and magnesium stearate are blended, passed through a No. 45 mesh U.S. sieve, and filled into hard gelatin capsules in 200 mg quantities.

Example 16

Suppositories each containing 225 mg of active ingredient are made as follows:

| | |
|---|---|
| 5-{[3,5-bis-(1,1-dimethylethyl)-4-hydroxyphenyl]methyl}-3-methyl-2-thioxo-4-thiazolidinone | 225 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

Example 17

Suspensions each containing 50 mg of medicament per 5 ml dose are made as follows:

| | |
|---|---|
| 4-{[3,5-bis(1,1-dimethylethyl)-4-hydroxyphenyl]methylene}-2-thioxo-1,3-oxathiolan-5-one | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to | 5 ml |

The medicament is passed through a No. 45 mesh U.S. sieve and mixed with the sodium carboxymethyl cellulose and syrup to form a smooth paste. The benzoic acid solution, flavor and color are diluted with some of the water and added, with stirring. Sufficient water is then added to produce the required volume.

Example 18

Capsules each containing 150 mg of medicament are made as follows:

| | |
|---|---|
| 5-{[3,5-bis(1,1-dimethylethyl)-4-hydroxyphenyl]methylene}-2-thiazolidinone | 150 mg |
| Starch | 164 mg |
| Microcrystalline cellulose | 164 mg |
| Magnesium stearate | 22 mg |
| Total | 500 mg |

The active ingredient, cellulose, starch and magnesium stearate are blended, passed through a No. 45 mesh U.S. sieve, and filled into hard gelatin capsules in 500 mg quantities.

**Claims for the Contracting States: BE CH DE FR GB IT LI LU NL SE**

1. A pharmaceutical formulation comprising as an active ingredient a compound of the formula I:

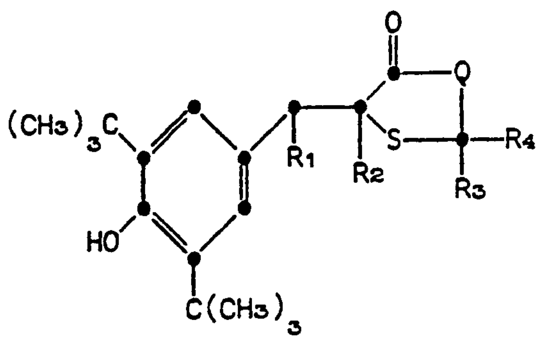

I

wherein

Q is O or NR, where R is hydrogen or $C_1$—$C_4$ alkyl;

$R_1$ and $R_2$ are each hydrogen, or when taken together form a bond; and

$R_3$ and $R_4$ are each hydrogen, or when taken together are =S, provided that when $R_1$ and $R_2$ are a bond,

Q is NR and R = H, then $R_3$ and $R_4$ are each H or a pharmaceutically-acceptable salt thereof, associated with one or more pharmaceutically-acceptable carriers or excipients therefor.

2. A pharmaceutical formulation as claimed in claim 1, wherein the active ingredient is 5-{[3,5-bis(1,1-dimethylethyl)-4-hydroxyphenyl]methylene}-3-methyl-4-thiazolidinone.

3. A compound of formula I, as defined in claim 1, for use in the treatment of inflammatory conditions.

4. A compound of formula III

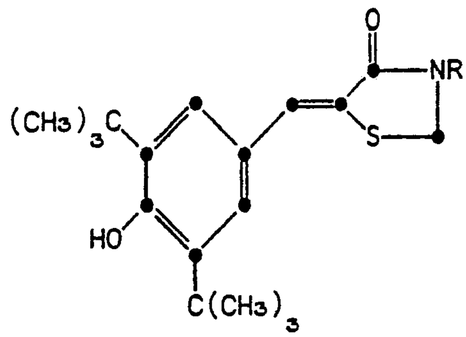

III

wherein R is hydrogen or $C_1$—$C_4$ alkyl, or a pharmaceutically-acceptable salt thereof, for use in preventing ischemia-induced cell damage in mammals.

5. 5-{[3,5-Bis(1,1-dimethylethyl)-4-hydroxyphenyl]methylene}-3-methyl-4-thiazolidinone for use in preventing ischemia-induced cell damage in mammals.

6. A compound of formula I as defined in claim 1, provided that when $R_1$ and $R_2$ taken together are a bond and Q is NH, $R_3$ and $R_4$ may only be hydrogen.

7. 5-{[3,5-bis(1,1-dimethylethyl)-4-hydroxyphenyl]methyl-4-thiazolidinone.

8. 5-{[3,5-bis(1,1-dimethylethyl)-4-hydroxyphenyl]methylene}-3-methyl-4-thiazolidinone.

9. A process for preparing a compound of formula I as claimed in claim 6 or 7, which comprises

(A) reacting a compound of formula:

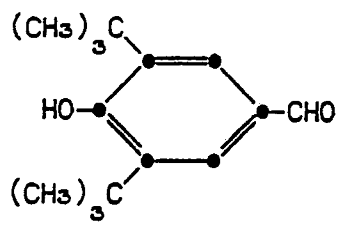

with a compound of formula

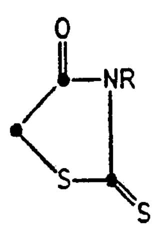

so as to provide a compound of formula I wherein $R_1$ and $R_2$ taken together represent a bond, Q is NR, and $R_3$ and $R_4$ taken together represent =S;

(B) reducing a compound of formula I wherein $R_3$ and $R_4$ represent =S so as to prepare a compound of formula I in which $R_3$ and $R_4$ are hydrogen;

(C) reducing a compound of formula I in which $R_1$ and $R_2$ represent a bond so as to prepare a compound of formula I in which $R_1$ and $R_2$ are hydrogen;

(D) alkylating a compound of formula I in which R is hydrogen so as to prepare a compound of formula I in which R is $C_1$—$C_4$ alkyl; or

(E) reacting a compound of formula

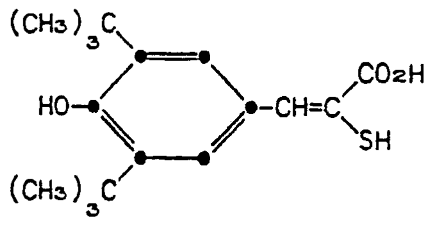

with

(i) formic acid so as to provide a compound of formula I wherein Q is O, $R_1$ and $R_2$ taken together represent a bond, and $R_3$ and $R_4$ represent hydrogen; or

(ii) carbon disulfide, so as tc provide a compound of formula I wherein Q is O, $R_1$ and $R_2$ taken together represent a bond, and $R_3$ and $R_4$ taken together represent =S.

**Claims for the Contracting State: AT**

1. A process for preparing a compound of the formula I:

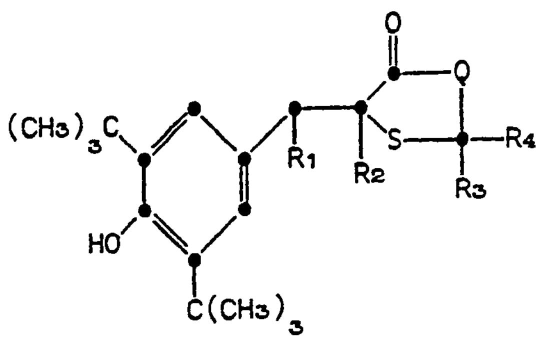

I

wherein

Q is O or Nr, where R is hydrogen or $C_1$—$C_4$ alkyl;

$R_1$ and $R_2$ are each hydrogen, or when taken together form a bond; and

$R_3$ and $R_4$ are each hydrogen, or when taken together are =S, provided that when $R_1$ and $R_2$ taken together are a bond and Q is NH, $R_3$ and $R_4$ may only be hydrogen, or a pharmaceutically-acceptable salt thereof, which comprises

(A) reacting a compound of formula:

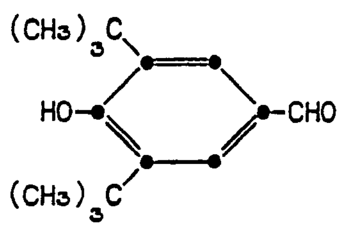

provided that when $R_1$ and $R_2$ are a bond, Q is NR and R is hydrogen, then $R_3$ and $R_4$ are each H, with a compound of formula

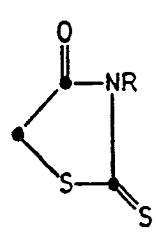

so as to provide a compound of formula I wherein $R_1$ and $R_2$ taken together represent a bond, Q is NR, and $R_3$ and $R_4$ taken together represent =S;

(B) reducing a compound of formula I wherein $R_3$ and $R_4$ represent =S so as to prepare a compound of formula I in which $R_3$ and $R_4$ are hydrogen;

(C) reducing a compound of formula I in which $R_1$ and $R_2$ represent a bond so as to prepare a compound of formula I in which $R_1$ and $R_2$ are hydrogen;

(D) alkylating a compound of formula I in which R is hydrogen so as to prepare a compound of formula I in which R is $C_1$—$C_4$ alkyl; or

(E) reacting a compound of formula

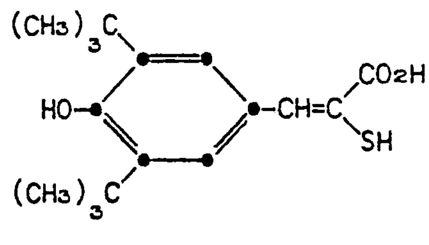

with

(i) formic acid so as to provide a compound of formula I wherein Q is O, $R_1$ and $R_2$ taken together represent a bond, and $R_3$ and $R_4$ represent hydrogen; or

(ii) carbon disulfide, so as to provide a compound of formula I wherein Q is O, $R_1$ and $R_2$ taken together represent a bond, and $R_3$ and $R_4$ taken together represent =S.

2. A process according to claim 1 for preparing a compound of formula III

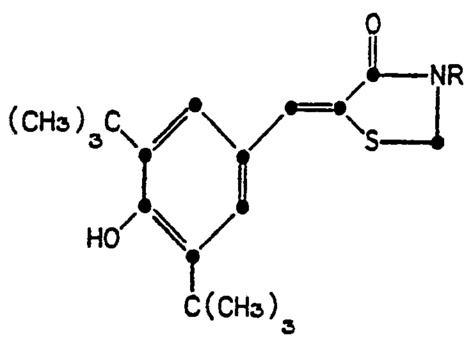

III

wherein R is hydrogen or $C_1$—$C_4$ alkyl, or a pharmaceutically-acceptable salt thereof.

3. A process according to claim 2 for preparing 5-{[3,5-bis(1,1-dimethylethyl)-4-hydroxyphenyl]methylene}-3-methyl-4-thiazolidinone.

4. A process according to claim 2 for preparing 5-{[3,5-bis(1,1-dimethylethyl)-4-hydroxyphenyl]methyl-4-thiazolidinone.

**Patentansprüche für die Vertragstaaten: BE CH DE FR GB IT LI LU NL SE**

1. Pharmazeutisches Präparat, enthaltend als Wirkstoff eine Verbindung der Formel I

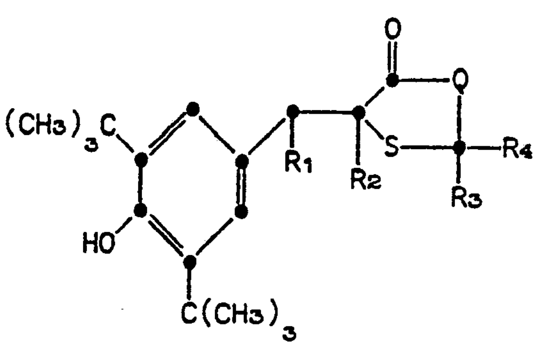

I

in der

Q die Bedeutung O oder NR hat, worin R Wasserstoff oder $C_1$—$C_4$-Alkyl bedeutet;

$R_1$ und $R_2$ jeweils Wasserstoff oder zusammen eine Bindung bedeuten; und

$R_3$ und $R_4$ jeweils Wasserstoff oder zusammen die Bedeutung =S haben, mit der Massgabe, dass wenn $R_1$ und $R_2$ eine Bindung bedeuten, Q die Bedeutung NR hat und R die Bedeutung Wasserstoff hat, $R_3$ und $R_4$ jeweils H sind,

sowie deren pharmazeutische verträgliche Salze, zusammen mit einem oder mehreren pharmazeutisch verträglichen Trägerstoffen oder Verdünnungsmitteln hierfür.

2. Pharmazeutisches Präparat nach Anspruch 1, wobei es sich beim Wirkstoff um 5-{[3,5-Bis-(1,1-dimethylethyl)-4-hydroxyphenyl]-methylen}-3-methyl-4-thiazolidinon handelt.

3. Verbindung der Formel I nach Anspruch 1 zur Verwendung bei der Behandlung von entzündlichen Zuständen.

4. Verbindung der Formel III

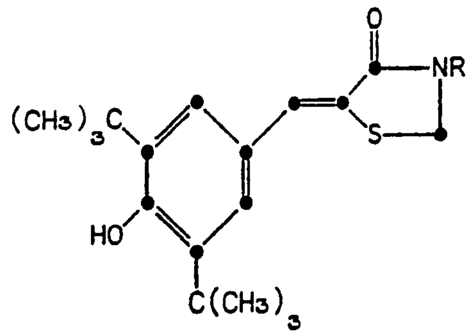

III

in der R Wasserstoff oder $C_1$—$C_4$-Alkyl bedeutet, oder ein pharmazeutisch verträgliches Salz davon zur Verwendung bei der Verhinderung von durch Ischämie induzierten Zellschädigungen bei Säugetieren.

5. 5-{[3,5-Bis-(1,1-dimethylethyl)-4-hydroxyphenyl]-methylen}-3-methyl-4-thiazolidinon zur Verwendung bei der Verhinderung von durch Ischämie induzierten Zellschädigungen bei Säugetieren.

6. Verbindung der Formel I nach Anspruch 1, mit der Massgabe, dass wenn $R_1$ und $R_2$ zusammen eine Bildung bedeuten und Q die Bedeutung NH hat, $R_3$ und $R_4$ nur die Bedeutung Wasserstoff haben können.

7. 5-{[3,5-Bis-(1,1-dimethylethyl)-4-hydroxyphenyl]-methyl}-4-thiazolidinon.

8. 5-{[3,5-Bis-(1,1-dimethylethyl)-4-hydroxyphenyl]-methylen}-3-methyl-4-thiazolidinon.

9. Verfahren zur Herstellung einer Verbindung der Formel I nach Anspruch 6 oder 7, umfassend

(A) Umsetzung einer Verbindung der Formel

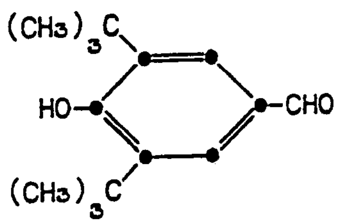

mit einer Verbindung der Formel

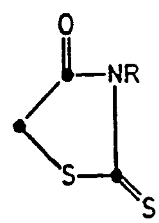

unter Bereitstellung einer Verbindung der Formel I, in der $R_1$ und $R_2$ zusammen eine Bindung darstellen, Q die Bedeutung NR hat und $R_3$ und $R_4$ zusammen die Bedeutung =S haben;

(B) Reduzieren einer Verbindung der Formel I, in der $R_3$ und $R_4$ die Bedeutung =S haben, unter Bildung einer Verbindung der Formel I, in der $R_3$ und $R_4$ Wasserstoff bedeuten;

(C) Reduzieren einer Verbindung der Formel I, in der $R_1$ und $R_2$ eine Bindung bedeuten, unter Herstellung einer Verbindung der Formel I, inder $R_1$ und $R_2$ Wasserstoff bedeuten;

(D) Alkylieren einer Verbindung der Formel I, in der R Wasserstoff bedeutet, under Herstellung einer Verbindung der Formel I, in der R die Bedeutung $C_1$—$C_4$-Alkyl hat; oder

(E) Umsetzen einer Verbindung der Formel

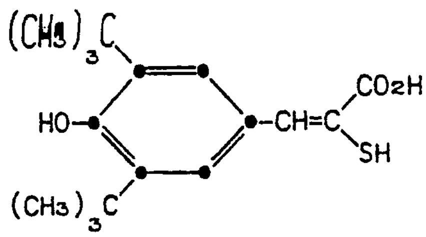

mit

(i) Ameisensäure, unter Bereitstellung einer Verbindung der Formel I, in der Q die Bedeutung O hat, $R_1$ und $R_2$ zusammen eine Bindung bedeuten und $R_3$ und $R_4$ Wasserstoff bedeuten; oder

(ii) Schwefelkohlenstoff unter Bereitstellung einer Verbindung der Formel I, in der Q die Bedeutung O hat, $R_1$ und $R_2$ zusammen eine Bindung bedeuten und $R_3$ und $R_4$ zusammen die Bedeutung =S haben.

**Patentansprüche für den Vertragstaat: AT**

1. Verfahren zur Herstellung einer Verbindung der Formel I

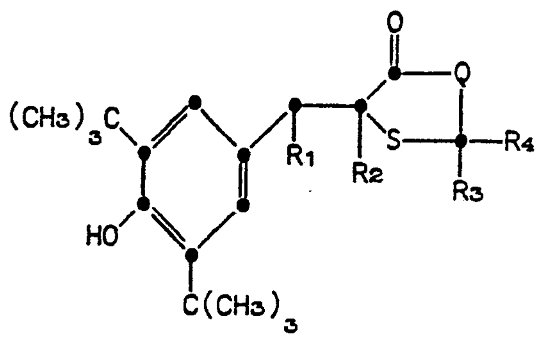

I

in der

Q die Bedeutung O oder NR hat, worin Wasserstoff oder $C_1$—$C_4$-Alkyl bedeutet;

$R_1$ und $R_2$ jeweils Wasserstoff oder zusammen eine Bindung bedeuten; und

$R_3$ und $R_4$ jeweils Wasserstoff oder zusammen die Bedeutung =S haben, mit der Massgabe, dass wenn $R_1$ und $R_2$ eine Bindung bedeuten, Q die Bedeutung NH hat und R die Bedeutung H hat, $R_3$ und $R_4$ jeweils H sind,

sowie von deren pharmazeutische verträglichen Salze, umfassend

(A) Umsetzung einer Verbindung der Formel

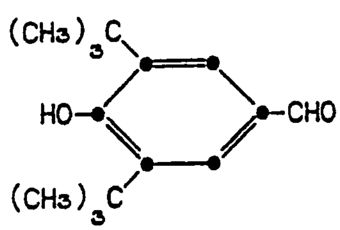

mit einer Verbindung der Formel

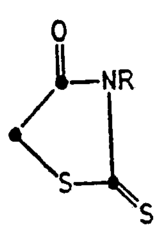

unter Bereitstellung einer Verbindung der Formel I, in der $R_1$ und $R_2$ zusammen eine Bindung darstellen, Q die Bedeutung NR hat und $R_3$ und $R_4$ zusammen die Bedeutung =S haben;

(B) Reduzieren einer Verbindung der Formel I, in der $R_3$ und $R_4$ die Bedeutung =S haben, unter Bildung

einer Verbindung der Formel I, in der $R_3$ und $R_4$ Wasserstoff bedeuten;

(C) Reduzieren einer Verbindung der Formel I, in der $R_1$ und $R_2$ eine Bindung bedeuten, unter Herstellung einer Verbindung der Formel I, in der $R_1$ und $R_2$ Wasserstoff bedeuten;

(D) Alkylieren einer Verbindung der Formel I, in der R Wasserstoff bedeutet, unter Herstellung einer Verbindung der Formel I, in der R die Bedeutung $C_1$—$C_4$-Alkyl hat; oder

(E) Umsetzen einer Verbindung der Formel

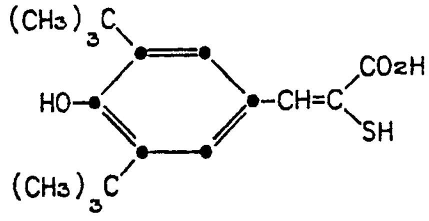

mit

(i) Ameisensäure, unter Bereitstellung einer Verbindung der Formel I, in der Q die Bedeutung O hat, $R_1$ und $R_2$ zusammen eine Bindung bedeuten und $R_3$ und $R_4$ Wasserstoff bedeuten; oder

(ii) Schwefelkohlenstoff unter Bereitstellung einer Verbindung der Formel I, in der Q die Bedeutung O hat, $R_1$ und $R_2$ zusammen eine Bindung bedeuten und $R_3$ und $R_4$ zusammen die Bedeutung =S haben.

2. Verfahren nach Anspruch 1 zur Herstellung einer Verbindung der Formel III

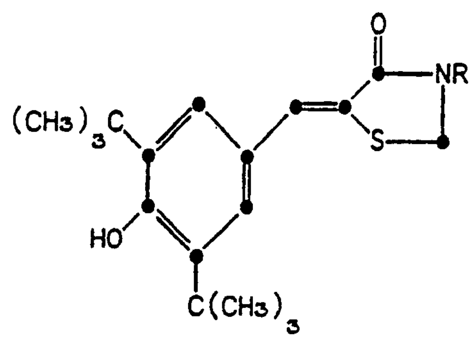

III

in der R Wasserstoff oder $C_1$—$C_4$-Alkyl bedeutet, oder eines pharmazeutisch verträglichen Salzes davon.

3. Verfahren nach Anspruch 2 zur Herstellung von 5-{[3,5-Bis-(1,1-dimethylethyl)-4-hydroxyphenyl]-methylen}-3-methyl-4-thiazolidinon.

4. Verfahren nach Anspruch 2 zur Herstellung von 5-{[3,5-Bis-(1,1-dimethylethyl)-4-hydroxyphenyl]-methyl}-thiazolidinon.

**Revendications pour les Etats contractants: BE CH DE FR GB IT LI LU NL SE**

1. Formulation pharmaceutique comprenant, comme ingrédient actif, un composé de formule I:

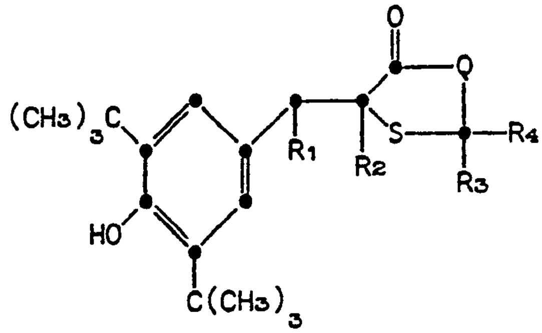

I

dans laquelle

Q représente O ou NR, où R représente un atome d'hydrogène ou un groupe alkyle en $C_1$—$C_4$;

$R_1$ et $R_2$ représentent chacun un atome d'hydrogène ou, lorsqu'ils sont pris ensemble, constituent une liaison; et

$R_3$ et $R_4$ représentent chacun un atome d'hydrogène ou, lorsqu'ils sont pris ensemble, représentent =S, avec cette réserve que, lorsque $R_1$ et $R_2$ représentent une liaison, Q représente NR et R = H, alors $R_3$ et

$R_4$ représentent chacun H, ou un de ses sels pharmaceutiquement acceptables, en association avec un ou plusieurs supports ou excipients pharmaceutiquement acceptables pour ce composé.

2. Formulation pharmaceutique selon la revendication 1, dans laquelle l'ingrédient actif est la 5-{[3,5-bis(1,1-diméthyléthyl)-4-hydroxyphényl]-méthylène}-3-méthyl-4-thiazolidinone.

3. Composé de formule I tel que défini dans la revendication 1, utile dans le traitement de conditions inflammatoires.

4. Composé de formule III:

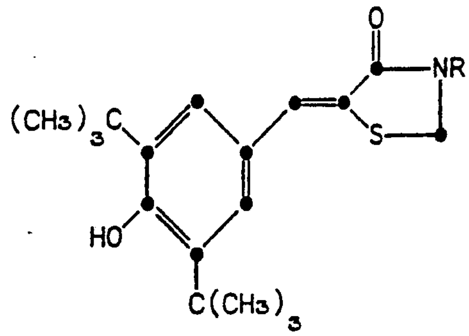

III

où R représente un atome d'hydrogène ou un groupe alkyle en $C_2$—$C_4$, ou un de ses sels pharmaceutiquement acceptables, utile pour la prévention de la dégradation cellulaire induite par ischémie, chez les mammifères.

5. 5-{[3,5-bis(1,1-diméthyléthyl)-4-hydroxyphényl]-méthylène}-3-méthyl-4-thiazolidinone utile pour la prevention de la dégradation cellulaire induite par ischémie, chez les mammifères.

6. Composé de formule I, tel que défini dans la revendication 1, avec cette réserve que, lorsque $R_1$ et $R_2$ pris ensemble forment une liaison et Q représente NH, $R_3$ et $R_4$ ne peuvent être qu'un atome d'hydrogène.

7. 5-{[3,5-bis(1,1-diméthyléthyl)-4-hydroxyphényl]-méthyl}-4-thiazolidinone.

8. 5-{[3,5-bis(1,1-diméthyléthyl)-4-hydroxyphényl]-méthylène}-3-méthyl-4-thiazolidinone.

9. Procédé de préparation d'un composé de formule I selon la revendication 6 ou 7, ce procédé consistant à:

(A) faire régir un composé de formule:

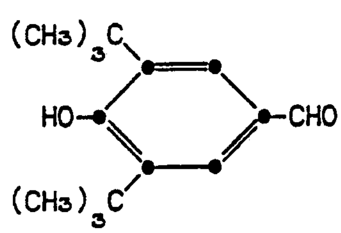

avec un composé de formule

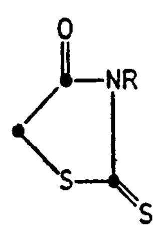

de façon à obtenir un composé de formule I, où $R_1$ et $R_2$ pris ensemble représentent une liaison, Q représente NR, et $R_3$ et $R_4$ pris ensemble représentent =S;

(B) réduire un composé de formule I, où $R_3$ et $R_4$ représentent =S, de façon à préparer un composé de formule I ou $R_3$ et $R_4$ représentent un atome d'hydrogène;

(C) réduire un composé de formule I, où $R_1$ et $R_2$ représentent une liaison, de façon à préparer un composé de formule I, où $R_1$ et $R_2$ représentent un atome d'hydrogène;

(D) alkyler un composé de formule I, où R représente un atome d'hydrogène, de façon à préparer un composé de formule I, où R représente un groupe alkyle en $C_1$—$C_4$; ou

(E) faire réagir un composé de formule

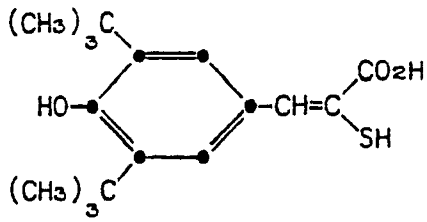

avec

(i) de l'acide formique, de façon à obtenir un composé de formule I, où Q représente O, $R_1$ et $R_2$ pris ensemble représentent une liaison, et $R_3$ et $R_4$ représentent un atome d'hydrogène; ou

(ii) du disulfuree de carbone, de façon à obtenir un composé de formule I, où Q représente O, $R_1$ et $R_2$ pris ensemble représentent une liaison, et $R_3$ et $R_4$ pris ensemble représentent =S.

**Revendications pour l'Etat contractant: AT**

1. Procédé de préparation d'un composé de formule I:

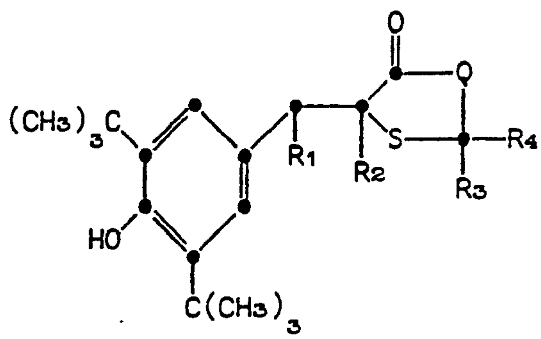

I

dans laquelle

Q représente O ou NR, où R représente un atome d'hydrogène ou un groupe alkyle en $C_1$—$C_4$;

$R_1$ et $R_2$ représentent chacun un atome d'hydrogène ou, lorsqu'ils sont pris ensemble, constituent une liaison; et

$R_3$ et $R_4$ représentent chacun un atome d'hydrogène ou, lorsqu'ils sont pris ensemble, représentent =S, avec cette réserve que, lorsque $R_1$ et $R_2$ constituent une liaison, Q représente NR et R représente un atome d'hydrogène, alors $R_3$ et $R_4$ représentent chacun H, et avec cette réserve que, lorsque $R_1$ et $R_2$ représentent une liaison, Q représente NH, $R_3$ et $R_4$ ne peuvent être qu'un atome d'hydrogène, ou un de ses sels pharmaceutiquement acceptables, ce procédé consistant à:

(A) faire réagir un composé de formule:

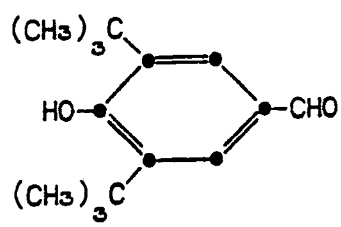

avec un composé de formule

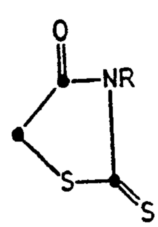

de façon à obtenir un composé de formule I, où $R_1$ et $R_2$ pris ensemble représentent une liaison, Q

représente NR, et $R_3$ et $R_4$ pris ensemble représentent =S;

(B) réduire un composé de formule I, où $R_3$ et $R_4$ représentent =S, de façon à préparer un composé de formule I ou $R_3$ et $R_4$ représentent un atome d'hydrogène;

(C) réduire un composé de formule I, où $R_1$ et $R_2$ représentent une liaison, de façon à préparer un composé de formule I, où $R_1$ et $R_2$ représentent un atome d'hydrogène;

(D) alkyler un composé de formule I, où R représente un atome d'hydrogène, de façon à préparer un composé de formule I, où R représente un groupe alkyle en $C_1$—$C_4$; ou

(E) faire réagir un composé de formule

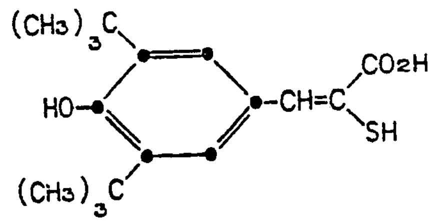

avec

(i) de l'acide formique, de façon à obtenir un composé de formule I, où Q représente O, $R_1$ et $R_2$ pris ensemble représentent une liaison, et $R_3$ et $R_4$ représentent un atome d'hydrogène; ou

(ii) du disulfure de carbone, de façon à obtenir un composé de formule I, où Q représente O, $R_1$ et $R_2$ pris ensemble représentent une liaison, et $R_3$ et $R_4$ pris ensemble représentent =S.

2. Procédé selon la revendication 1 pour la préparation d'un composé de formule III

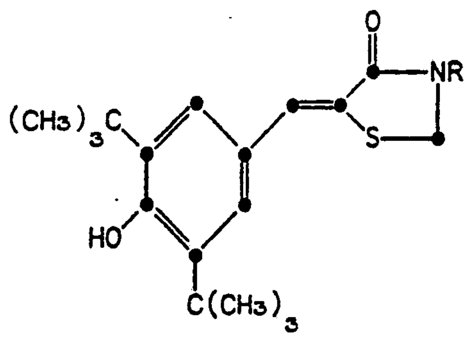

III

ou R représente un atome d'hydrogène ou un groupe alkyle en $C_1$—$C_4$, ou un de ses sels pharmaceutiquement.

3. Procédé selon la revendication 2, pour la préparation de la 5-{[3,5-bis(1,1-diméthyléthyl)-4-hydroxyphényl]-méthylène}-3-méthyl-4-thiazolidinone.

4. Procédé selon la revendication 2, pour la préparation de la 5-{[3,5-bis(1,1-diméthyléthyl)-4-hydroxyphényl]-méthyl}-4-thiazolidinone.